(19) Europäisches Patentamt
European Patent Office
Office européen des brevets

(11) Veröffentlichungsnummer: **0 236 981 B1**

# EUROPÄISCHE PATENTSCHRIFT

(12)

(45) Veröffentlichungstag der Patentschrift: **10.04.91**

(51) Int. Cl.⁵: **C07C 31/10**, C07C 29/74

(21) Anmeldenummer: **87103233.0**

(22) Anmeldetag: **06.03.87**

(54) **Verfahren zum Deodorieren von Isopropylalkohol.**

(30) Priorität: **12.03.86 DE 3608210**

(43) Veröffentlichungstag der Anmeldung:
**16.09.87 Patentblatt 87/38**

(45) Bekanntmachung des Hinweises auf die
Patenterteilung:
**10.04.91 Patentblatt 91/15**

(84) Benannte Vertragsstaaten:
**AT BE CH DE ES FR GB GR IT LI LU NL SE**

(56) Entgegenhaltungen:
**DE-A- 2 840 118    DE-B- 1 056 110
DE-C- 1 667 190    GB-A- 687 380
US-A- 2 584 325    US-A- 2 666 735
US-A- 2 696 508    US-A- 2 729 682
US-A- 2 857 436**

(73) Patentinhaber: **RWE-DEA Aktiengesellschaft
für Mineraloel und Chemie
Überseering 40
W-2000 Hamburg 60(DE)**

(72) Erfinder: **Osterburg, Günther
Ahornstrasse 5
W-4137 Rheurdt 2(DE)**
Erfinder: **Gluzek, Karl-Heinz
Die Schraag 11
W-4234 Alpen 1(DE)**
Erfinder: **Reith, Wolfgang
St.-Nikolaus Strasse 23
W-4170 Geldern-Walbeck(DE)**
Erfinder: **Neier, Wilhelm
An der Landwehr 40
W-4134 Rheinberg 3(DE)**

(74) Vertreter: **Schupfner, Gerhard D.
Müller, Schupfner & Gauger Karlstrasse 5
Postfach 14 27
W-2110 Buchholz/Nordheide(DE)**

EP 0 236 981 B1

## Beschreibung

Die Erfindung betrifft ein Verfahren zum Deodorieren von Isopropylalkohol, der durch katalytische Hydratation von Propen hergestellt worden ist, durch Inkontaktbringen mit einem Festbett-Deodorierungsharz.

Isopropylalkohol wird insbesondere durch Umsetzung von Propen mittels Schwefelsäure oder durch Direkthydratation an sulfonierten Kationenaustauscherharzen hergestellt. Er enthält Spuren von verschiedenen Verbindungen, welche dem technischen Produkt einen unangenehmen Geruch verleihen, der dieses für die industrielle Weiterverarbeitung und insbesondere für den Einsatz in der kosmetischen und pharmazeutischen Industrie ungeeignet macht.

Die als Verursacher des Geruchs anzusehenden Verunreini. gungen konnten nur zum Teil durch gaschromatographische Untersuchungen nachgewiesen werden. Ihr Gehalt in dem technischen Alkohol liegt im ppb-Bereich und darunter. Es handelt sich hierbei insbesondere um organische Schwefelverbindungen, wie Schwefelwasserstoff, Carbonylsulfid und Mercaptane. Bisher waren aufwendige Behandlungsverfahren erforderlich, wie Destillations- und Adsorptionsverfahren, um den technischen Alkohol von diesen Substanzen zu befreien. So wurde über viele Jahre eine Deodorierung durch Behandlung mit Aktivkohle vorgenommen, wobei die Aktivkohle in regelmäßigen Zeitabständen durch kostenaufwendige Dampfbehandlung regeneriert werden mußte. Zudem eignen sich nur wenige Kohlen für eine solche Behandlung, da sie entweder zu viel Nebenprodukte, im wesentlichen Aceton, erzeugen, nicht oder nur beschränkt regenerierbar oder nicht hinreichend wirksam zur Deodorierung sind.

Aus der US-PS 2 857 436 ist ein Verfahren bekannt, wonach niedere technische Alkohole zur Geruchsverbesserung mit einem feinteiligen Kiesel/Eisen-Material von hoher Oberfläche in Berührung gebracht werden. Nach einer weiteren Ausführungsform des Verfahrens dieser Patentschrift werden die niederen Alkohole zur Geruchsverbesserung mit unglasiertem Porzellan und Stahlwolle in Berührung gebracht.

Gemäß der US-PS 2 729 682 wird versucht, eine Geruchsverbesserung von Isopropylalkohol dadurch herbeizuführen, daß man bei der Herstellung dem Propylenstrom $C_4$- bis $C_6$-Monoolefine hinzugibt, mit Schwefelsäure umsetzt und das Umsetzungsprodukt hydrolisiert und hierbei gleichzeitig das darin enthaltene $C_4$-$C_6$-Olefin zusammen mit den Verunreinigungen, die den Geruch verursachen, bei Temperaturen bis 300°C zu hochsiedenden Verunreinigungen umsetzt. Der gereinigte Isopropylalkohol wird sodann durch extraktive Destillation mit Wasser gewonnen.

Gemäß der deutschen Offenlegungsschrift 28 40 118 wird eine Deodorierung von $C_2$- und $C_3$-Alkoholen in Gegenwart einer großen Zahl hydrieraktiver Metalle, insbesondere von Nickel- und Platinmetallen, auf anorganischen Trägermaterialien bei erhöhter Temperatur durchgeführt.

In der deutschen Patentschrift 16 67 190 ist ein Verfahren zur Reinigung von Alkoholen unter Entfernung von darin enthaltenen Eisencarbonylverbindungen offenbart. Dort läßt man den Alkohol durch ein Bett aus Kationenaustauscherharzen fließen, die mit hydrieraktiven Metall-Ionen beladen sind. Die Metall-Ionen müssen jeweils in ihrer höchsten Oxydationsstufe vorliegen. Geeignet sind zahlreiche hydrieraktive Metalle, wie Eisen(III)-, Silber(I)-, Kupfer(II)-, Gold(III)-, Cer(IV)-, Thallium(III)-Ionen. Der Kationenaustauscher dient dort als Träger des RedoxKatalysators und als Fänger für das freiwerdende Eisen(II).

Die Verfahren des Standes der Technik sind entweder umständlich und kostenaufwendig, oder aber es entstehen bei der dort vorgeschlagenen Behandlungsweise weitere Verunreinigungen, insbesondere Ketone und Äther.

Der Erfindung liegt daher die Aufgabe zugrunde, ein Deodorierungsverfahren bereitzustellen, das den unangenehmen Geruch des technischen Produktes weitgehend nimmt, einfach zu handhaben und kostengünstig ist und das nicht seinerseits Nebenprodukte wie insbesondere Ketone und Äther bildet.

Diese Aufgabe wird erfindungsgemäß dadurch gelöst, daß das Deodorierungsharz hergestellt worden ist, indem man ein starksaures Kationenaustauscherharz entweder vollständig mit Silber in Ionenform belädt oder vor oder nach der Silberbeladung mit Alkali- oder Erdalkalihydroxid, vorzugsweise mit Natriumhydroxid, praktisch vollständig neutralisiert.

Überraschenderweise wurde somit gefunden, daß ein mit Silber in Ionenform beladenes und neutralisiertes Kationenaustauscherharz zur Deodorierung von Isopropylalkohol der durch katalytische Hydratation von Propen hergestellt worden ist, vorzüglich geeignet ist. Die erfindungsgemäße Deodorierung erfolgt durch Überleitung des Alkohols über das Festbett-Deodorierungsharz bei Atmosphärendruck und Umgebungstemperatur. Höhere Drücke können angewendet werden, sind aber nicht erforderlich.

Erhöhte Temperaturen sind ohne weiteres insbesondere dann möglich, wenn das Deodorierungsharz in praktisch vollständig neutralisierter Form vorliegt. Man führt die Deodorierung im allgemeinen bei 0° bis 60°C, insbesondere bei 10 bis 50°C und vorzugsweise bei 20 bis 30°C durch.

2

Da die Deodorierung sehr schnell verläuft, sind hohe Belastungen des Festbett-Deodorierungsharzes, so von 0,1 bis 15 l Alkohol / 1 Kationenaustrauscherharz und Stunde möglich. Vorzugsweise kann man 0,4 bis 10 l Alkohol / 1 Kationenaustauscherharz und Stunde durchsetzen.

Die Silberbeladung beträgt vorzugsweise 4 bis 10 g (berechnet als Metall) je Liter Kationenaustauscherharz und insbesondere 5 bis 8 g (berechnet als Metall) je Liter Kationenaustauscherharz.

Es wird eine vollständige Beladung des starksauren Kationenaustauscherharzes angestrebt, um saure Kontaktstellen weitgehend zu vermeiden. Diese führen zu Nebenreaktionen, und zwar insbesondere zur Ätherbildung, was unerwünscht ist.

Gemäß der bevorzugten Ausführungsform des erfindungsgemäßen Verfahrens wird das Kationenaustauscherharz zunächst vollständig neutralisiert, indem man vor oder nach der Silberbeladung das Kationenaustauscherharz mit Alkali-oder Erdalkalihydroxid, insbesondere mit Natriumhydroxid, praktisch vollständig neutralisiert. Überraschenderweise konnten vorneutralisierte starksaure Kationenaustauscher, beispielsweise in der Natrium-Form, direkt mit Silberionen beladen werden.

Die vollständige Neutralisation des Kationenaustauscherharzes mit Ag-Ionen ist möglich, jedoch aus wirtschaftlichen Gründen nicht zweckmäßig. Es empfiehlt sich, gemäß der obigen alternativen Ausführungsform das Kationenaustauscherharz zunächst mit billigen Alkali- oder Erdalkalikationen und insbesondere mit Natriumkationen praktisch vollständig zu beladen und dann das vorneutralisierte Harz mit Silberionen zu behandeln und hierdurch Natrium- gegen Silberionen auszutauschen.

Bei den als Trägerharz eingesetzten starksauren Kationenaustauschern handelt es sich um solche auf Kunstharzbasis, insbesondere um Styrol-Divinylbenzol-Mischpolymerisate mit Sulfonsäuregruppen am aromatischen Kern, die auch zur Herstellung des Isopropylalkohols aus Propen durch Direkthydratation zum Einsatz kommen. Bevorzugt handelt es sich um Harze vom Typ Amberlyst ® 15 der Firma Rohm & Haas Co., U.S.A. oder LEWATIT ® SPC 118 der Firma Bayer AG, Leverkusen.

Das verbrauchte Deodorierungsharz kann unter Rückgewinnung des Metalls regeneriert und wieder eingesetzt werden.

Das erfindungsgemäße Verfahren ist sowohl zur Deodorierung des trockenen Isopropylalkohols wie auch zur Deodorierung von Gemischen des Alkohols mit Wasser und insbesondere azeotropen Isopropylalkohols geeignet.

Die folgenden Beispiele erläutern die Erfindung.

Beispiel 1 (Vergleichsbeispiel)

Ein kommerzieller starksaurer Kationenaustauscher, Amberlyst ® 15 der Firma Rohm & Haas, wurde durch Behandlung mit wäßriger Silbernitrat-Lösung mit 7,6 g Silber (berechnet als Metall)/l Harz beladen (entsprechend einer Belegung der Kapazität des Katalysators von 3,9%) und in der sauren Form zur Deodorierung von Isopropylalkohol eingesetzt.

Dazu wurden über eine Säule von 28 cm Länge und 2,6 cm Durchmesser mit 0,15 l silberbeladenes Harz bei 50° C 24 Stunden lang 0,065 l Isopropylalkohol/h von oben nach unten durchgesetzt.

Die gaschromatographische Untersuchung einer nach 24 Stunden Laufzeit entnommene Probe Isopropylalkohol zeigte gegenüber dem zur Deodorierung eingesetzten Alkohol folgende Zunahme an Verunreinigungen:

Niedere Kohlenwasserstoffe    40 ppm

Diisopropylether    390 ppm

Die Geruchsprüfung ergab, daß die vor der Deodorierung enthaltenen Geruchsträger weitgehend entfernt waren, daß aber ein neuer Geruch nach Diisopropylether erkennbar war.

Beispiel 2

Ein kommerzieller starksaurer Kationenaustauscher, Amberlyst ® 15 der Firma Rohm & Haas, wurde zunächst durch Behandlung mit wäßriger Silbernitrat -Lösung mit 7,6 g Silber (berechnet als Metall)/l Harz beladen. Danach wurden die noch freien starksauren Gruppen des Kationenaustauschers durch Zugabe von 1 n Natriumhydroxid-Lösung vollständig neutralisiert und das neutral gewaschene Harz zur Deodorierung von Isopropylalkohol eingesetzt.

Die Deodorierungsbedingungen entsprachen denen von Beispiel 1.

Die gaschromatographische Untersuchung einer nach 24 Stunden Laufzeit entnommenen Probe Isopropylalkohol zeigte gegenüber dem zur Deodorierung eingesetzten Alkohol keine Veränderung.

Die Geruchsprüfung ergab, daß die vor der Deodorierung enthaltenen Geruchsträger weitgehend entfernt waren und kein neuer Nebengeruch entstanden war.

Beispiel 3

Ein kommerzieller starksaurer Kationenaustauscher, LEWATIT ® SPC 118 der Firma Bayer AG, wurde zunächst durch Behandlung mit wäßriger Silbernitrat-Lösung mit 8 g Silber (berechnet als Metall)/l Harz beladen, dann durch Zugabe von 1 n Natriumhydroxid-Lösung vollständig neutralisiert und danach zur Deodorierung von Isopropylalkohol eingesetzt.

Dazu wurden über eine Säule von 100 cm Länge und 21 cm Durchmesser mit 35 l silberbeladenes Harz bei einer mittleren Temperatur von 22 °C in 88 Tagen 422,4 m³ Isopropylalkohol, das sind 0,2 m³/h bzw. 5,7 l Isopropylalkohol/l Harz, durchgesetzt. Die Deodorierungsleistung des eingesetzten Harzes war danach noch nicht erschöpft.

Die dem Isopropylalkohol vor der Deodorierung anhaftenden Geruchsträger wurden während des gesamten Zeitraumes gleichbleibend weitgehend entfernt. In keiner Zeit konnte eine Verschlechterung der Isopropylalkoholqualität durch neue Verunreinigungen oder Gerüche beobachtet werden.

Beiepiel 4

Ein kommerzieller starksaurer Kationenaustauscher vom Typ Amberlyst ® 15 der Firma Rohm & Haas wurde in seiner inaktiven neutralen $Na^+$-Form durch Behandlung mit wäßriger Silbernitrat-Lösung mit 6 g Silber (berechnet als Metall)/l Harz belegt und danach zur Deodorierung von Isopropylalkohol eingesetzt.

Über eine Deodorierungssäule mit insgesamt 5 m³ des wie vorn beschriebenen Deodorierungsharzes wurden in 643 Tagen insgesamt 226.388 m³ Isopropylalkohol, das sind im Mittel ca. 15 m³ Alkohol/h bzw. 3 Volumenteile Alkohol/Volumenteil Deodorierungsharz, bei 20 °C von oben nach unten durchgesetzt. Der Spitzendurchsatz betrug während dieser Zeit 20 m³/h.

Die Deodorierungsleistung des Harzes war nach dieser Zeit noch nicht erschöpft. Die Geruchsbeurteilung des deodorierten Isopropylalkohols war während des gesamten Zeitraumes gut. Neue Gerüche oder Verunreinigungen wurden nicht erkannt.

**Ansprüche**

1. Verfahren zum Deodorieren von Isopropylalkohol, der durch katalytische Hydratation von Propen hergestellt worden ist, durch Inkontaktbringen mit einem Festbett-Deodorierungsharz,
   **dadurch gekennzeichnet,**
   daß das Deodorierungsharz hergestellt worden ist, indem man ein starksaures Kationenaustauscherharz entweder vollständig mit Silber in Ionenform belädt oder vor oder nach der Silberbeladung mit Alkali- oder Erdalkalihydroxid, vorzugsweise mit Natriumhydroxid, praktisch vollständig neutralisiert.

2. Verfahren nach Anspruch 1,
   **dadurch gekennzeichnet,**
   daß man das Kationenaustauscherharz mit Silberionen in einer Menge von 1 bis 10 g, vorzugsweise 5 bis 8 g (berechnet als Metall) je Liter Harz belädt.

3. Verfahren nach Anspruch 1 oder Anspruch 2,
   **dadurch gekennzeichnet,**
   daß man 0,4 bis 10 Liter Alkohol / Liter Harz und Stunde in Kontakt bringt.

4. Verfahren nach einem der vorhergehenden Ansprüche,
   **dadurch gekennzeichnet,**
   daß man die Deodorierung bei 10 bis 50 °C und vorzugsweise bei 20 bis 30 °C durchführt.

## Claims

1. A process for deodorizing isopropyl alcohol which has been produced by catalytic hydration of propene which comprises contacting said isopropyl alcohol with a fixed-bed deodorizing resin wherein said resin has been prepared by completely impregnating a strongly acidic cation exchange resin with silver in ionic form or, prior to or subsequent to the impregnation with silver, by practically completely neutralizing said resin with an alkali- or alkaline earth hydroxide, preferably sodium hydroxide.

2. A process according to claim 1, in which said cation exchange resin contains from about 1 to 10 grams of silver ions (calculated as metal), preferably from 5 to 8 grams, per liter of said cation exchange resin.

3. A process according to claim 1 or 2, in which the deodorization of isopropyl alcohol is conducted at a rate from about 0.4 to 10 liters of isopropyl alcohol per liter of said cation exchange resin and hour.

4. A process according to any one of the preceding claims, in which said deodorization is conducted at a temperature ranging from about 10 to 50°C, preferably from about 20 to 30°C.

## Revendications

1. Procédé de désodorisation d'alcool isopropylique produit par l'hydratation catalytique de propène, en mettant l'alcool en contact avec une résine désodorisante disposée sous forme de lit fixe, caractérisé en ce qu'on prépare la résine désodorisante en imprégnant en totalité une résine échangeuse de cations fortement acide d'ions d'argent, ou en neutralisant en quasi-totalité une telle résine échangeuse, avant ou après l'imprégnation d'argent, avec de l'hydroxyde alcalin ou alcalino-terreux, de préférence de l'hydroxyde de sodium.

2. Procédé selon la revendication 1, caractérisé en ce qu'on dépose sur l'échangeur de cations 1 à 10 grammes d'ions d'argent (exprimé en métal), de préférence 5 à 8 grammes, par litre de résine.

3. Procédé selon la revendication 1 ou 2, caractérisé en ce qu'on réalise la désodorisation en mettant en contact 0,4 à 10 litres d'alcool par litre de résine et par heure.

4. Procédé selon l'une des revendications précédentes, caractérisé en ce qu'on réalise la désodorisation à 10 - 50°C, de préférence à 20 - 30°C.